# EUROPEAN PATENT APPLICATION

(11) **EP 3 628 310 A1**
(43) Date of publication of application: **01.04.2020**
(21) Application number: 18196664.9
(22) Date of filing: 25.09.2018
(51) Int. Cl.: A61K 9/20, A61K 9/14, A61K 9/16, A61K 31/7076

(54) **PHARMACEUTICAL COMPOSITION COMPRISING SOLID DISPERSIONS OF AMORPHOUS CLADRIBINE AND PHARMACEUTICALLY ACCEPTABLE WATER SOLUBLE CARRIER**

(71) Applicant: Synbias Pharma AG, 8200 Schaffhausen (CH)
(72) Inventor: ROST, Michael, 370 05 Ceské Budejovice (CZ); REHÁCKOVÁ, zdenka, 370 01 Nové Homole (CZ); ZABUDKIN, Oleksandr, 76593 Gernsbach (DE); MATHA, Vladimir, 373 82 Borsov nad Vltavou (CZ)
(74) Representative: Berger, Axel Bernhard

(57) **Abstract**

The present invention relates to a pharmaceutical composition comprising a solid dispersion of amorphous cladribine and a water-soluble pharmaceutically acceptable carrier, as well as a method for preparing same.

## Description

The present invention relates to a pharmaceutical composition comprising a solid dispersion of amorphous cladribine and a water-soluble carrier. The water-soluble carrier may be in particular spray-dried high amylose pea maltodextrin. The pharmaceutical composition may be used as a medicament, particularly in the treatment of multiple sclerosis.

### Background of the Present Invention

Cladribine (2-CdA), chemically 2-chloro-2'-deoxyadenosine, of formula 1 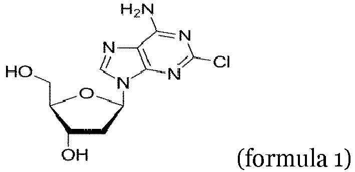 is an antineoplastic agent used in the treatment of lymphoproliferative diseases including hairy cell leukemia (HCL), acute myeloid leukemia (AML), chronic lymphocytic leukemia (CLL), Langerhans cell histiocytosis, non-Hodgkin's lymphomas, Waldenstrom macroglobulinemia, and relapsing remitting multiple sclerosis. 2-CdA is a prodrug that must be converted into its respective nucleotide as an essential requirement for cytotoxicity. Triphosphate analog inhibits various processes involved in DNA and RNA synthesis. Moreover, the effect of nucleoside analog in leukemic cells may involve modulation of apoptosis pathways, cell-cycle control, or signal transduction pathways.

Cladribine is available both in injectable and oral forms. However, the physical-chemical features of Cladribine make it a rather difficult compound to formulate oral final dosage forms. First, it is only slightly water soluble at room temperature. As such, its oral absorption is dissolution-rate limited and it requires enhancement in solubility and dissolution rate for increasing its oral bioavailability. Second, it is sensitive to low pH environments. At acidic pH, decomposition markedly increases with time, even at physiological temperatures. At pH 2 and 37°, after 6 h, only 13% of 2-CdA remains in solution; at pH 1 after 2 h of heating to 37°C 2-CdA content is as low as 2%; the calculated half time T_{1/2} is equal to 1.6 h (pH 2, 37°C) and 0.37 h (pH 1, 37°C) (Tarasiuk A, Skierski J, Kazimierczuk Z, Stability of 2-chloro-2'-deoxyadenosine at various pH and temperature. Arch Immunol Ther Exp (Warsz). 1994;42(1):13-5).

Accordingly, both the low solubility and the low pH stability of cladribine represent a significant complication for the development of oral dosage forms.

Major obstacles associated with the successful development of cladribine oral dosage forms represent a combination of a) their narrow absorption window in the upper small intestine, where cladribine enters tissues via nucleoside transporter proteins distributed in the epithelial lining of the intestine and undergoes intracellular phosphorylation to the active form, 2-CdA triphosphate, by deoxycytidine kinase in the target cells, and b) degradation by low pH and by specific enzymes, e.g. PNP, and UP, produced by microbial microflora.

Hence, if the drug is not quickly released in the upper small intestine area, it exhibits a low bioavailability and undergoes fast degradation; a concern which makes its release profile critical.

Solubility and dissolution rate are the two important parameters to be considered for achieving the therapeutic effectiveness of the drug through the oral administration. Poor aqueous solubility and dissolution profile of insoluble drugs remain the major problem in the formulation design and development of oral dosage forms.

Various techniques have been used in attempt to improve solubility and dissolution rates of poorly soluble drugs including solid dispersion (SD), micronization, lipid-based formulations, liquisolid compacts and complexation. SD and complexation with hydrophilic carriers have been demonstrated as a promising technique for improving the solubility and dissolution of poorly soluble drugs. The improved solubility and dissolution profiles of SD and complexation formulation could be explained by the particle size reduction, the change of drug from crystalline to the amorphous form, solubilizing effect of hydrophilic carriers and better wettability of drugs surrounded by carriers.

Lipophilic drug-hydrophilic carrier complexes, commonly known as inclusion complexes, can be prepared simply by adding the drug and excipients together, resulting in enhanced drug solubilization. Inclusion complexes are formed by the insertion of the non-polar molecule (known as guest) into the cavity of another molecule (as host). The most commonly used host molecules are cyclodextrins.

Cyclodextrin complexation is advantageous because of low hygroscopicity, low toxicity, high fluidity, excellent compatibility and compressibility of cyclodextrin complexation, improving the stability of drugs in a formulation and resulting in longer shelf life.

Cyclodextrins are cyclic oligosaccharides with a hydrophilic outer surface and a hydrophobic central cavity. This molecular structure gives cyclodextrins some unique properties. The hydrophilic exterior of the molecule can make them water soluble, while the hydrophobic cavity provides a micro-environment for appropriate sized non-polar molecules. In aqueous solutions, cyclodextrins are capable of forming inclusion complexes with many drugs by taking up a whole drug molecule, or rather some non-polar part of the molecule, into the cavity.

The term "solid dispersion" refers to a group of solid products comprising at least two different components, generally a hydrophilic matrix and a hydrophobic drug.

Various preparation methods for solid dispersion have been reported in the literature including solvent evaporation, kneading, melting or fusion method, melt extrusion, coprecipitation, co-evaporation, spray drying, gel entrapment technique, supercritical fluid technology, lyophilization technique.

When referring to a dispersion of the components such that the system is chemically and physically uniform or homogenous throughout or consists of one phase (as defined in thermodynamics), solid dispersion will be called a "solid solution" or a "glassy solution". These systems do not contain any significant amounts of active ingredients in their crystalline or microcrystalline state, as evidenced by thermal analysis (DSC) or X-ray diffraction analysis (WAXS).

One of the underlying principles of formulations of solid dispersions is the generation of the amorphous state which is considered to be more soluble than the crystalline state because in the amorphous state, no energy is required to break the crystal lattice found in the crystalline phase.

In US patent 6,194,395, Schultz and Naeff used 2-Hydroxypropyl-β-cyclodextrin (HPBCD) to improve both cladribine water solubility and low pH sensitivity.

Later, Bodor and Dandiker in US patent 8,785,415 described a formation of an amorphous cladribine-cyclodextrin complex as an intimate amorphous admixture of amorphous inclusion complex of cladribine with an amorphous cyclodextrin and amorphous free cladribine associated with amorphous cyclodextrin as a non-inclusion complex-amorphous free cladribine associated with amorphous cyclodextrin as a non-inclusion complex, and its effect on cladribine oral absorption.

Bodor and Dandiker described just one method of preparing cladribine-cyclodextrin complex, which is freeze drying. The formulation according to US 8,785,415 requires the manipulation with high volumes of water and long complexation time due to limited cladribine solubility. Preparation of the final dosage form requires costly, equipment demanding, high energy, time consuming processes.

In fact, the final dosage form prepared according to the above mentioned patent represents a mixture of complexed and adsorbed API in a ratio of 30-40% to 70-60%, respectively, which makes the control and predictability of the process rather difficult and could influence solubilization and decomposition of the API in the stomach followed by lower 2-CdA absorption. In addition, there is no direct evidence about the positive effect of 30-40% 2-CdA in inclusion complex over the rest of adsorbed molecules on the surface of the carrier.

Thus, in view of the prior art cited above, there is still a need for alternative, improved pharmaceutical compositions comprising amorphous cladribine, which are stable, easier and cheaper to produce, and which are suitable for use on a commercial scale. In view of this, it would be desirable to produce stable amorphous cladribine and to find a robust process for making such a stable amorphous cladribine.

### Brief Description of the Invention

The present invention relates to a pharmaceutical composition comprising a solid dispersion of amorphous cladribine and a water-soluble pharmaceutically acceptable carrier.

It has been found that a solid dispersion of amorphous cladribine and a water-soluble carrier provides for high stability at low pH values and high bioavailability.

The water-soluble pharmaceutically acceptable carrier may be spray-dried high amylose pea maltodextrin. Spray-dried high amylose pea maltodextrin may be obtained by partial hydrolyses of pea starch as a carrier. This carrier offers a new technical approach to provide a stable solid dispersion with amorphous cladribine suitable for the formulation of oral dosage forms. The solid dispersion enhances the overall drug release properties of the poorly soluble drug cladribine.

The weight ratio of cladribine to the water-soluble pharmaceutically acceptable carrier in the solid dispersion may be at least 1:3, preferably at least 1:4.

The weight ratio of cladribine to the water-soluble pharmaceutically acceptable carrier may be in the range of 1:3 to 1:20, preferably 1:4 to 1:15, more preferably 1:5 to 1:15, most preferably 1:5 to 1:10.

The solid dispersion may comprise a residual solvent content of less than about 10%, based on the weight of the solid dispersion, preferably less than about 9%, more preferably less than about 7%, further preferably less than about 5%, even further preferably less than about 2%.

The pharmaceutical composition may further comprise one or more pharmaceutically acceptable excipients.

The pharmaceutical composition may be provided in the form of a tablet or capsule.

Said pharmaceutical composition may be used as a medicament, particularly in the treatment of multiple sclerosis.

Further, use of the pharmaceutical composition for oral and/or parenteral administration is described.

The present invention also relates to a method for preparing a solid dispersion of amorphous cladribine and a water-soluble pharmaceutically acceptable carrier, comprising the steps:
- preparing a solution comprising cladribine and a water-soluble pharmaceutically acceptable carrier in a solvent or in a mixture of solvents, and
- removing the solvent from the solution, preferably by means of vacuum evaporation, spray drying, or freeze drying.

Cladribine may be provided in crystalline, polymorphic or amorphous form and dissolved in the solvent or mixture of solvents for preparing the solution.

The water-soluble pharmaceutically acceptable carrier may be spray-dried high amylose pea maltodextrin. The weight ratio of cladribine to the water-soluble pharmaceutically acceptable carrier in the solution may be at least 1:3, preferably at least 1:4.

The weight ratio of cladribine to the water-soluble pharmaceutically acceptable carrier in the solution may be in the range of 1:3 to 1:20, preferably 1:4 to 1:15, more preferably 1:5 to 1:15, most preferably 1:5 to 1:10.

The solution may be prepared with at least one solvent selected from water and a C1-C4 alcohol, preferably water, ethanol, methanol, and isopropanol, and mixtures thereof, more preferably the solvent comprises a mixture of water and ethanol or a mixture of water and isopropanol.

The solvent may comprise water in an amount of at least 70% w/w of the total solvent.

The solvent may be a hydroalcoholic solution.

After removing the solvent or mixture of solvents from the solution the obtained solid may be dried. The dried solid may comprise a residual solvent content of less than about 10%, based on the weight of the solid dispersion, preferably less than about 9%, more preferably less than about 7%, further preferably less than about 5%, even further preferably less than about 2%.

### Brief Description of the Drawings

In the following, preferred embodiments of the invention are described with reference to the figures, in which:
- **Figures 1 a,b**: show XRD diffraction patterns of cladribine-linecaps complexes according to embodiments of the present invention and references samples;
- **Figure 2**: shows the behaviour of cladribine API and solid dispersion in a HCl buffer of pH 1.4.

### Detailed Description of the Invention

In the following, embodiments and variations according to the present invention are described in more detail. It is, however, emphasized that the present invention is not limited to these embodiments and variations. It is also mentioned that in the following only individual embodiments of the invention can be described in more detail. The skilled person will realize, however, that the features described in relation to these specific embodiments can be combined in a different manner within the scope of the invention, and that individual features may also be omitted if these seem dispensable in a given case.

The present invention relates to a pharmaceutical composition comprising a solid dispersion of amorphous cladribine and a pharmaceutically acceptable carrier.

In yet another aspect there are provided methods for preparing a solid dispersion comprising amorphous cladribine, according to one embodiment comprising at least one of:
a. providing a solution containing cladribine and a pharmaceutically acceptable carrier in a solvent or a mixture of solvents;
b. removing the solvent from the solution of a);
c. optionally drying a solid formed in b) to afford the desired amorphous solid dispersion of cladribine.

Cladribine in an amorphous premix form of the present application is sufficiently stable and well suited for use in pharmaceutical formulations, which are useful in the treatment of disease, including, but not limited to, multiple sclerosis.

In a further embodiment, there is provided a method for preparing amorphous cladribine, comprising removing solvent from a solution of cladribine and a pharmaceutically acceptable carrier. In an embodiment, a method for preparing a solid dispersion containing amorphous cladribine comprises removing solvent from a solution of cladribine in combination with a pharmaceutically acceptable carrier.

A solution of cladribine may be provided by dissolving cladribine in a solvent or a mixture of solvents. Any polymorphic form may be used in the preparation of solution, such as crystalline forms including solvates and hydrates.

In the scope of finding potentially new cladribine solubilizers suitable for commercial production of amorphous cladribine, spray dried high amylose pea maltodextrin, available as Kleptose® Linecaps, was tested.

Kleptose® Linecaps (maltodextrin from pea starch) is rich in linear and soluble amylose. This amylose fraction enables the formation of inclusion compounds as required for taste masking and other applications. Amylose exists as helical structures, and like a cyclodextrin these bear a hydrophilic external surface and a hydrophobic internal cavity, created by the presence of glycosidic ether bonds, forming a cavity which potentially enables amylose to encapsulate certain drugs.

The encapsulation potential of amylose is also able to improve the cold-water solubility of certain drug molecules. However, in order to meet the solubilisation capacity of cyclodextrins, higher quantities in weight (about 10-fold more) of Kleptose® Linecaps are needed, this is due to its higher molecular weight. On a molar scale, Kleptose® Linecaps and cyclodextrins have comparable effects.

In agreement with the above information Kleptose Linecaps should solubilize by forming inclusion complex CLD only in high Linecaps : API ratio. In case of cladribine the ratio should be close to 1:100.

Surprisingly, when Linecaps was diluted in hydroalcoholic solution its solubilizing effect improved unexpectably. It was found that the use of hydroalcoholic solution results in a significant reduction of pea maltodextrin needed to solubilize and stabilize cladribine in a solution and after evaporation provides stable amorphous solid dispersion suitable to formulate oral dosage form with acceptable stability and dissolution profile.

The present invention is based on the finding that the combination of spray-dried high amylose pea maltodextrin obtained by partial hydrolyses of pea starch as a carrier and hydroalcoholic solution offers new technical approach to stable solid dispersion with amorphous cladribine API suitable for formulation of oral dosage forms.

Cladribine and Kleptose Linecaps may be dissolved either in the same solvent or they may be dissolved in different solvents and then combined to form a mixture. In embodiments, the solid dispersion described herein includes cladribine and the carrier present in weight ratios ranging from about 1:3 to about 1:15, preferably from 1:5 to 1:10.

Solvents which may be used for dissolving cladribine and the carrier include, but are not limited to mixture of water, and organic solvents like C1-C4 alcohols. Specific examples of organic solvents that may be utilized for the present invention include ethanol, methanol, isopropanol and mixtures thereof. The quantity of solvent used for dissolution depends on the solvent and the dissolution temperature adopted.

Linecaps is not well soluble in water: EtOH solution. When Linecaps is solubilized in water, followed by addition of EtOH up to 1:1 (v/v), it remained soluble and solubilized cladribine remains stable for days.

The dissolution temperatures may range from about 35°C to about 60°C, depending on the solvent used for solubilization. Any other temperature is also acceptable as long as clear solutions are provided.

Optionally, the solution obtained above may be filtered to remove any undissolved particles before further processing. The undissolved particles may be removed suitably by filtration, centrifugation, decantation, and other techniques. The solution may be filtered by passing through paper, glass fiber, or other membrane material.

Removal of the solvent may be carried out suitably using techniques such as atmospheric evaporation or evaporation under vacuum. Suitable techniques which may be used for solvent removal include vacuum evaporation, spray drying, and freeze drying (lyophilization).

Evaporation of the solvent may be conducted under a vacuum, such as below about 100 mm Hg, or below about 600 mm Hg, at temperatures such as about -20°C to about 70°C. Any temperature and vacuum conditions may be used as long as there is no increase in the impurity levels of the product.

For example, spray drying, and evaporation by Buchi Rotavapor are more suitable for industrial scale production with a batch size of about 100 g or about 1 Kg, or greater. According to the present invention the obtained amorphous form by spray drying or Buchi Rotavapor is quickly dissolved from pharmaceutical compositions.

The amorphous material obtained may be collected from the equipment using techniques such as by scraping, or using techniques specific to the particular apparatus optionally under nitrogen atmosphere.

Optionally, drying of solid product may be carried out under suitable conditions to afford the desired solid dispersion of cladribine in an amorphous form, substantially free of residual solvents. In embodiments of the present invention, a solid dispersion of cladribine contains residual solvents greater than about 1 % and less than about 10% with respect to the weight of the solid dispersion. In a particular embodiment, a solid dispersion has a residual solvent content less than about 2% by weight. In another embodiment, a solid dispersion has a residual solvent content ranging from about 4% to about 7%, by weight.

Drying may be carried out until the residual solvent content reduces to a desired amount, such as an amount that is within the limits given by the International Conference on Harmonisation of Technical Requirements for Registration of Pharmaceuticals for Human Use ("ICH") guidelines. The guideline solvent level depends on the type of solvent but is not more than about 5000 ppm, or about 4000 ppm, or about 3000 ppm.

The drying may be carried out at reduced pressures, such as below about 650 mm Hg, or below about 50 mm Hg, at temperatures such as about 35°C to about 70°C. The drying may be carried out for any desired time period that achieves the desired result, such as times about 1 to 20 hours, or longer. Drying may also be carried out for shorter or longer periods of time depending on the product specifications.

Drying may be suitably carried out in equipment such as a tray dryer, vacuum oven, air oven, or using a fluidized bed drier, and the like.

Cladribine and its impurities may be analyzed using HPLC.

The present invention still further provides a method to prepare pharmaceutical compositions comprising anamorphous cladribine and one or more pharmaceutically acceptable excipients. The method may comprise mixing or granulating the amorphous solid dispersion cladribine with one or more pharmaceutically acceptable excipients, followed by tableting or encapsulation, using equipment and methods well-known to the skilled artisan.

The pharmaceutical compositions of the present invention display dissolution behaviour typical for immediate-release formulations. During preparation and storage of the pharmaceutical compositions of the present invention remain in the amorphous form.

The pharmaceutical compositions of the present invention may be packaged in blister pack material. The blister pack materials to be used in accordance with the present invention may be any blister pack material known to a person of ordinary skill in the art. Suitable blister pack materials to be used in accordance with the present invention are selected from the group of Aclar, PVC/Alu, Duplex/Alu, Triplex/Alu and Alu/Alu. To ensure protection of the compositions of the present invention from e.g. moisture and thereby preventing polymorphic conversions, Aclar, Triplex/Alu and Alu/Alu are particularly preferred blister pack materials.

The pharmaceutical composition in accordance with the present invention may be used as a medicament. The pharmaceutical composition typically may be used in the treatment of multiple sclerosis.

The present invention is illustrated by the following Examples.

### Examples

### Example 1: Cladribine:Linecaps with a weight ratio 1:10

5.0 g Linecaps was dissolved in 25 ml of distilled water under stirring conditions at room temperature, followed by addition of 10 ml of ethanol. 0.5 g cladribine was suspended in the Linecaps:water:ethanol solution and heated up to 50° C. Once the solution was clear and the spray-drier was preheated, the solution was pumped into the spray-drier, where the solvent was instantaneously evaporated and a solid product was collected.

An analogous process was performed using isopropanol instead of ethanol.

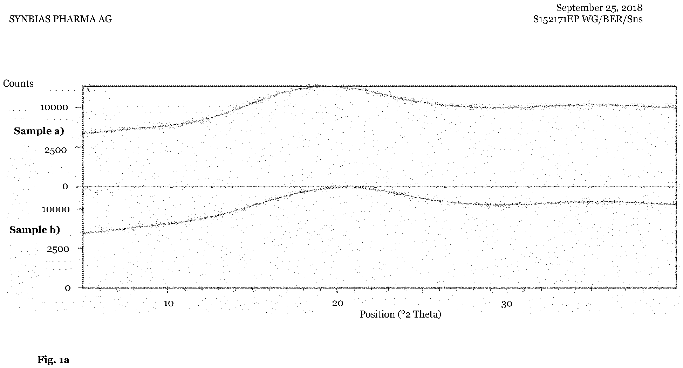

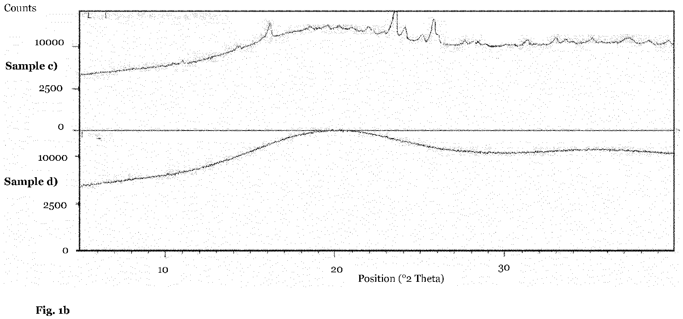

XRPD data of the samples were recorded (**Fig. 1**). Reference sample a) refers to the linecaps powder as such, which was used for the above described preparation of cladribine:linecaps. Sample b) refers to a cladribine:linecaps solid dispersion in a weight ration of 1:10 in water/ethanol, as described above. Reference sample c) refers to a physical mixture of cladribine and linecaps in a weight ratio of 1:10, i.e. not being prepared as a solid dispersion of amorphous cladribine. Sample d) refers to cladribine linecaps solid dispersion with a weight ratio of 1:10 in water/isopropanol, as described above.

The XRD data of sample b) and sample d) show that the isolated solid dispersion product was fully amorphous. The same results were achieved when vacuum evaporation or spray drying were applied for drying/solvent removal (not shown).

In comparison, sample c), presenting a physical mixture of cladribine and linecaps shows that the cladribine compound is at least partially in its crystalline form.

### Example 2: Behaviour of cladribine API and solid dispersion in HCl buffer pH 1.4

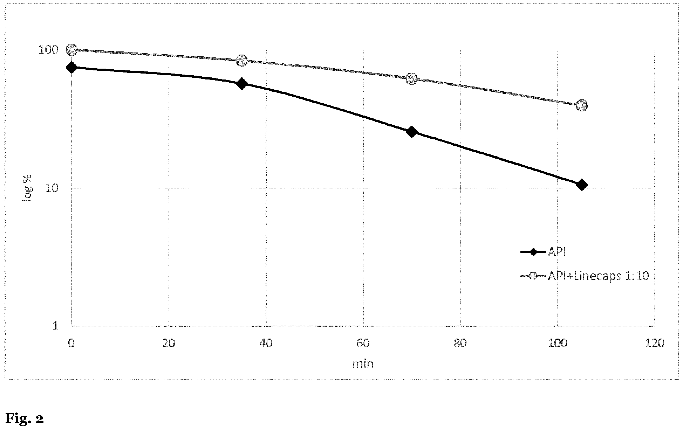

The effect of the solid dispersion on cladribine stability was tested at pH 1.4 and temperature 37°C.
10 mg of cladribine API (reference sample) or 10 mg cladribine solid dispersion according to the present invention, e.g. prepared as described above in Example 1, were solubilized in HCl buffer pH 1.4. The time when solid dispersion was completely dissolved was considered timepoint 0. Both samples were inserted in an autosampler and cladribine concentrations were determined for the time period of 105 min. As evident from Figure 2 cladribine solid dispersion was significantly more stable at pH 1.4 than the pure cladribine API. When expressed by relative comparison of areas, concentration of cladribine solid dispersion was about 75% more than that of cladribine after 105 min. Area under curve was calculated according to trapezoidal rule.

### Example 3: Cladribine tablets

Cladribine tablets were prepared by direct compression. 110.0 grams of cladribine linecaps 1:10 solid dispersion (55% total weight) according to the present invention, e.g. as prepared in Example 1, was sieved and subsequently mixed with 78.0 grams of microcrystalline cellulose (39% total weight) and 10 grams of sodium croscarmellose (5% total weight), followed by addition of 2 grams of magnesium stearate (1% total weight).

The powder blend obtained was tableted in 200 mg tablets, containing 10 mg of cladribine. The measured dissolution profiles in a buffer of pH 4.5 is presented in the table below:

| Sample: | % of dissolution | | | |
|---|---|---|---|---|
| | 0 [min] | 10 [min] | 15 [min] | 20 [min] |
| Tablet 1 | 0.0 | 100.8 | 100.6 | 100.8 |
| Tablet 2 | 0.0 | 100.5 | 100.4 | 100.6 |
| Tablet 3 | 0.0 | 100.2 | 100.3 | 100.4 |
| Tablet 4 | 0.0 | 100.6 | 100.8 | 100.9 |

The results indicate that the tested tablets dissolved fast, reached 100% dissolution in 10 min, and fully complied with dissolution requirement for fast dissolving products.

## Claims

1. Pharmaceutical composition comprising a solid dispersion of amorphous cladribine and a water-soluble pharmaceutically acceptable carrier.

2. The pharmaceutical composition according to claim 1, where the water-soluble pharmaceutically acceptable carrier is spray-dried high amylose pea maltodextrin.

3. The pharmaceutical composition according to any one of the preceding claims, wherein the weight ratio of cladribine to the water-soluble pharmaceutically acceptable carrier in the solid dispersion is at least 1:3, preferably at least 1:4.

4. The pharmaceutical composition according to claim 3, wherein the weight ratio of cladribine to the water-soluble pharmaceutically acceptable carrier is in the range of 1:3 to 1:20, preferably 1:4 to 1:15, more preferably 1:5 to 1:15, most preferably 1:5 to 1:10.

5. The pharmaceutical composition according to any one of the preceding claims, wherein the solid dispersion comprises a residual solvent content of less than about 10%, based on the weight of the solid dispersion, preferably less than about 9%, more preferably less than about 7%, further preferably less than about 5%, even further preferably less than about 2%.

6. The pharmaceutical composition according to any one of the preceding claims additionally comprising one or more pharmaceutically acceptable excipients.

7. The pharmaceutical composition according to any one of the preceding claims in the form of a tablet or capsule.

8. Pharmaceutical composition according to any one of the preceding claims for use in oral and/or parenteral administration.

9. A method for preparing a solid dispersion of amorphous cladribine and a water-soluble pharmaceutically acceptable carrier, comprising the steps of:
- preparing a solution comprising cladribine and a water-soluble pharmaceutically acceptable carrier in a solvent or in a mixture of solvents, and
- removing the solvent from the solution, preferably by means of vacuum evaporation, spray drying, or freeze drying.

10. The method of claim 9, wherein the water-soluble pharmaceutically acceptable carrier is spray-dried high amylose pea maltodextrin.

11. The method of claim 9 or 10, wherein cladribine is provided in crystalline, polymorphic or amorphous form and dissolved in the solvent or mixture of solvents for preparing the solution.

12. The method of any one of claims 9 to 11, wherein the solution is prepared with at least one solvent selected from water and a C1-C4 alcohol, preferably wherein the solvent is selected from water, ethanol, methanol, and isopropanol, and mixtures thereof, more preferably the solvent comprises a mixture of water and ethanol or a mixture of water and isopropanol.

13. The method of any one of claims 9 to 12, wherein the solvent comprises water in an amount of at least 50%, preferably at least 60%, more preferably at least 70% w/w of the total solvent.

14. The method of any one of claims 9 to 13, wherein after removing the solvent or mixture of solvents from the solution the obtained solid is dried.

15. Solid dispersion of amorphous cladribine and a water-soluble pharmaceutically acceptable carrier as obtained by one of the claims 9 to 14.
